# EUROPÄISCHE PATENTSCHRIFT

(11) **EP 0 745 666 B1**
(45) Veröffentlichungstag und Bekanntmachung des Hinweises auf die Patenterteilung: **24.07.2002**
(21) Anmeldenummer: 96106257.7
(22) Anmeldetag: 20.04.1996
(51) Int. Cl.: C12M 1/06, C12M 1/04

(54) **Bioreaktor**
Bioreactor
Bioréacteur

(30) Priorität: 03.06.1995 DE 19520485
(43) Veröffentlichungstag der Anmeldung: 04.12.1996
(73) Patentinhaber: Degussa AG, 40474 Düsseldorf (DE)
(72) Erfinder: Schilling, Bernhard, 38118 Braunschweig (DE); Pfefferle, Walter, Dr., D-33790 Halle/Westfalen (DE); Bachmann, Bernd, Dr., 33824 Werther (DE); Leuchtenberger, Wolfgang, Prof. Dr., 33619 Bielefeld (DE); Deckwer, Prof. Dr. W.-D., 26129 Oldenburg (DE)

(56) Entgegenhaltungen:
- DE-A- 4 242 425
- FR-A- 1 563 722
- FR-A- 2 088 354
- FR-A- 2 336 476
- US-A- 3 251 749

## Beschreibung

Die Erfindung betrifft für Labor- und Versuchszwecke vorgesehene Rührreaktoren, die sich aufgrund spezieller Einbauten dafür eignen, die unter ihrer Verwendung erhaltenen Ergebnisse auf den technischen Maßstab zu übertragen.

Im allgemeinen werden chemische ebenso wie mikrobielle oder enzymatische Prozesse auf ihre Eignung für die Überführung in den kommerziellen Maßstab im Labormaßstab vorgetestet.

Mikroorganismen z. B. werden in kleinen Reaktoren angezogen, die möglichst günstige Voraussetzungen für Wachstum und Produktbildung garantieren.

Unter solchen Bedingungen findet man eine weitgehend gleichmäßige Verteilung der Nährstoffe im Reaktor bei gleichzeitig optimal ablaufenden Transportmechanismen zwischen Nähstofflösung und Mikroorganismen.

Einen wesentlichen Faktor stellen bei aeroben Prozessen immer auch Eintrag und Verteilung des Sauerstoffs in die Fermentationsbrühe dar. Aufgrund der Möglichkeiten, seine Ausgestaltung zu variieren, findet der Rührreaktor auf dem Gebiet biotechnologischer Verfahren sehr häufig seine Anwendung.

Hat sich die Leistungsfähigkeit eines Mikroorganismus' im Labormaßstab als aussichtsreich erwiesen, zeigt die Praxis, daß die Übertragung dieses Verfahrens bzw. seiner Ergebnisse in den Produktionsmaßstab mit einer Vielfalt von Problemen verbunden ist.
Der Fachmann weiß, daß in dieser Größenordnung nicht dieselbe Leistungsfähigkeit eines Stammes und damit dieselbe Produktmenge wie im Labormaßstab erwartet werden darf.
Ein Reaktor dieser Bauart kann nicht schlicht in seiner Größenordnung um den Faktor X vergrößert werden, wenn man die X-fache Produktmenge erhalten will.

Zu dieser Problematik gibt es daher eine Vielzahl von Untersuchungen, die unter bestimmten, gegebenen Voraussetzungen Lösungsansätze zu bieten versuchen.

Die im Stand der Technik enthaltenen Angaben zur Reaktorauslegung und Maßstabübertragung weichen jedoch zum Teil auch aufgrund der unterschiedlichen Meßverfahren stark voneinander ab.

Beispielhaft zu nennen sind Untersuchungen des Scale up-Verhaltens durch mathematische Modelle (NAGY et al., Computers Chem. Engng. Vol. 18, Suppl. 663 bis 667, 1994).

Auf der anderen Seite werden Reaktorsysteme beschrieben, die die Verhältnisse aus dem technischen Maßstab durch Rührkesselkaskaden simulieren.

So verwenden OOSTERHUIS et al., Biotechn. Letters Vol. 5, No. 3, 141 bis 146 (1983) zwei hintereinandergeschaltete Reaktoren, in denen definierte unterschiedliche Bedingungen (z. B. sauerstoffreiche und sauerstoffarme Kulturbedingung) herrschen.

Die Zellen der Mikroorganismen durchlaufen bei diesem Versuchsablauf verschiedene, genau definierte Zustände. Auf diesem Wege wird die Zahl der zu messenden Zustände jedoch auf die Anzahl der Reaktoren begrenzt. Übergangszustände werden kaum erfaßt, und auch das Problem der Rückvermischung vernachlässigt.

Es besteht daher auch weiterhin das ungelöste Problem, Reaktoren als Testsysteme für das Scale up-Verhalten von Fermentationsprozessen und enzymatischen Verfahren bereitzustellen.

Aus FR-A-1 563 722, FR-A-2 336 476 oder der DE 42 42 425 A sind Rührreaktoren mit mindestens einem Scheibenrührer und mindestens einer horizontal angebrachten lochscheibe bekannt.

Es wurde nun gefunden, daß man einen Laborfermenter (Rührreaktoren) u. a. im Hinblick auf den relevanten Parameter Mischzeit θ₉₀ durch Einbauten so abändern kann, daß er, bei vergleichbarem volumenbezogenen Leistungseintrag, einem Reaktor von 10 bis zu >100 m³ entspricht, der diese Einbauten nicht enthält.

Gegenstand der Erfindung ist ein Laborfermenter (Rührreaktor), der mit Scheibenrührern ausgerüstet ist, und in dem mindestens eine Lochscheibe ober- und/oder unterhalb des oder der Rührblätter angebracht ist.
Das Verhältnis von Lochoberfläche zur Scheibenoberfläche beläuft sich auf 10 bis 40 %, insbesondere auf ca 20 %. Die Scheibenoberfläche deckt bevorzugt 60 bis 90 % , insbesondere 75 bis 85 % des Reaktorquerschnitts ab.

Dabei kann die Zahl der Löcher beliebig gewählt werden, insbesondere liegt sie zwischen und 12, wobei die Anordnung der Löcher vorteilhaft möglichst symmetrisch erfolgt.

Sind zwei oder mehr Scheibenrührer auf der Rührachse montiert, befindet sich im allgemeinen eine Lochscheibe, bevorzugt mittig befestigt, zwischen den Rührern.

Unter besonderen Bedingungen werden aber auch zwei gleichoder unterschiedlich gelochte Scheiben zwischen den Scheibenrührern angebracht, deren Zahl sich z. B. bei einer Höhe des Rührreaktors von 0,75 m auf 2 bis 10 beläuft.

Eine bevorzugte Variante besteht darin, daß die Lochscheiben an der Wand oder an vertikalen Gewindestangen befestigt sind, natürlich ohne Behinderung der Rührachse. Auf diesem Weg wird der eventuelle Aufstieg von Gasblasen an der Reaktorwand verhindert. Es ist auch möglich, die Lochscheiben alternierend an der Rührachse oder der Reaktorwand zu befestigen.

Mit Hilfe der horizontalen Einbauten (Lochscheiben) gelingt es, den Stoffaustausch in vertikaler Richtung in einem Laborreaktor mit 10 bis 500 l Inhalt so zu reduzieren, wie es den Verhältnissen in einem für die Produktion eingesetzten Reaktor von >10m³ entspricht.
Nach EINSELE et al. (Chem. Rundschau (1976), 25, 53 bis 55) liegt die Mischzeit θ 95 von Reaktoren dieser Größe bei ca. 100 s.

Unter Mischzeit wird nach dieser Definition diejenige Zeit verstanden, welche notwendig ist, um nach der Zugabe einer gegebenen Menge Säure oder Lauge 95 % des pH-Endwertes zu erhalten.
Da sich jedoch gezeigt hat, daß der Wert von 95 % nur schwierig zu ermitteln ist, wird in der vorliegenden Anmeldung der 90 %-Wert gemessen, ohne daß dadurch die Aussagekraft der von EINSELE beschriebenen Methode geschwächt wird.

Diese, das Durchmischungsverhalten beschreibende Größe kann in Laborfermentern mit den erfindungsgemäß eingebauten Lochscheiben problemlos ermittelt werden.

Voraussetzung für einen sinnvollen Vergleich bildet natürlich eine geometrisch ähnliche Gestalt der Reaktoren. Das Volumen der zum Test verwendeten Reaktoren liegt im Rahmen der üblicherweise im Labor verwendeten Vorrichtungen, also von 10 bis 500 l, wobei diese Angaben nicht als ausschließlich zu werten sind.
Mit den erfindungsgemäß abgewandelten Standardreaktoren gelingt es, ein realistisches Bild davon zu gewinnen, welchen Einfluß die Mischzeit z. B. auf die Fermentation hat.
Die Mischzeit kann z. B. auf dem Wege der Fluoreszens-, pHoder Leitfähigkeits-methode ermittelt werden.
Es zeigt sich, daß man unter Verwendung der erfindungsgemäß abgewandelten Standardreaktoren auf einfachem Weg für das Scale up, z. B. von Fermentationsprozessen aussagefähige Informationen über Stoffumsätze und Biomassen erhält, die man nach dem Stand der Technik nur unter Berücksichtigung vieler Vorgaben zu ermitteln versucht und trotzdem nur mit einem großen Gehalt an Unsicherheit erhält.

Die Angaben über die Mischzeit sind insbesondere unter Angabe des sogenannten Leistungseintrags von Nutzen. Dieser liegt im allgemeinen bei enzymatischen Reaktionen und Fermentations-prozessen bei 1 bis 4 kW/m³.
Dabei wird die Leerlaufleistung des Rührers zuvor abgezogen. Mit diesem Leistungseintrag sind in den erfindungsgemäßen Rührreaktoren Mischzeiten θ₉₀ von 40 bis 300 sec, in Abhängigkeit von der Zahl der Einbauten nachzustellen.

Eine Variante der Charakterisierung des Verhaltens von Modellreaktoren besteht auch in der Angabe des "Arbeitspunktes", der sich durch seine Lage im Koordinatenssystem θ₉₀ gegen Leistungseintrag definiert.

### Beispielteil

Es wurden Fermentationsversuche mit L-Lysin produzierenden Stämmen durchgeführt, ohne daß die Maßstabsübertragung jedoch auf diese zu beschränken ist.
Ein Standardreaktor (40 l, idealer Rührkessel) war mit 4 Strombrechern und 2 Scheibenrührern mit 0,1 m Scheibendurchmesser, 0,02 m Blatthöhe, auf 1/3 und 2/3 der Flüssigkeitshöhe montiert, ausgerüstet.
In den abgeänderten Standardreaktor waren 6 Scheibenrührer eingebaut, die im Abstand von 7 cm auf der Rührwelle montiert wurden. Zwischen die Rührblätter wurden 5 Lochscheiben nach außen gegen die Reaktorwand abgedichtet, mit einem Lochdurchmesser von 0,11 m montiert (Abstand Rührer Lochscheibe 3,5 cm). (Verhältnis Lochfläche : Scheibenfläche 1:5 = 20%).

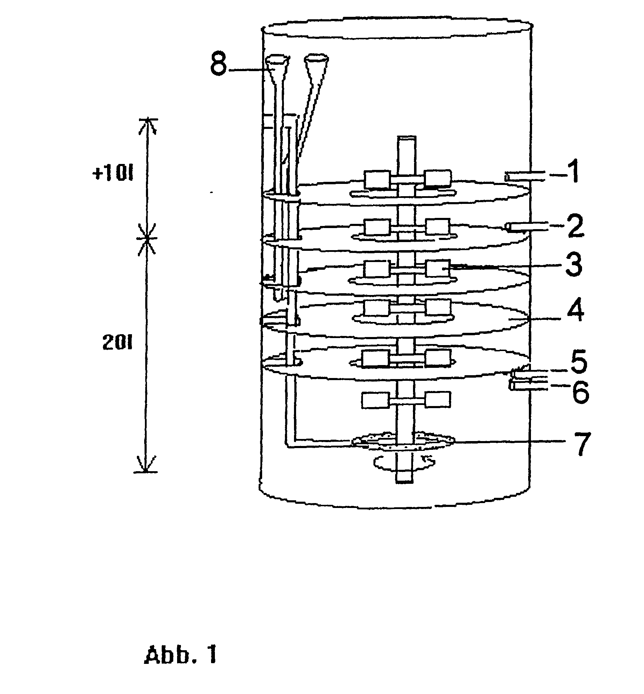

Die Abbildung 1 gibt die erfindungsgemäße Gestaltung des Versuchsreaktors (Lochscheiben) ebenso wie für die zur Durchführung der Mischzeitmessung und Fermentation notwendigen Einbauten wieder.
Mit Hilfe der pH-Methode (EINSELE s.o.) wurde die Mischzeit 90 bei 20 l Füllung, 400 upm und 2 vvm Belüftung bestimmt. Als Mischzeiten ergaben sich für den Standardreaktor 10 s und für den erfindungsgemäß abgeänderten Reaktor 130 s, so daß sich daraus für Reaktoren von ca. 100 m³ und mehr entsprechende Versuchsparameter ableiten lassen.
Wie die anschließenden Versuche zeigen, findet man z. B. deutliche Einflüsse auf die Stoffumsätze und die Menge der gebildeten Biomasse.

### Beispiel 1

12 Zwei-Liter-Kolben wurden mit 360 ml des folgenden Schüttelkolbenmediums befüllt und 20 min bei 121 °C sterilisiert. Nach Abkühlen wurden die Kolben mit dem leucinauxotrophen Lysinproduktionsstamm corynebacterium glutamicum DSM5715 beimpft und 24 h bei 150 rpm und 30 °C inkubiert. Die Kulturbrühe von je 6 Kolben wurde vereinigt und als Inokulum für jeweils einen Fermenter bereitgestellt.

| Schüttelkolbenmedium: | |
|---|---|
| Saccharose | 20 g/kg |
| Melasse | 43,6 g/kg |
| Sojamehlhydrolysat | 120 ml/kg |
| Hefeextrakt | 0,5 g/kg |
| (NH₄)₂SO₄ | 32 g/kg |
| Harnstoff | 6,0 g/kg |
| MgSO₄.7H₂O | 0,25 g/kg |
| KH₂PO₄ | 0,5 g/kg |
| Citronensäure.H₂O | 0,6 g/kg |
| FeSO₄.7H₂O | 10 mg/kg |
| MnSO₄.H₂O | 10 mg/kg |
| L-Leucin | 60 mg/kg |
| L-Threonin | 40 mg/kg |
| L-Methionin | 80 mg/kg |
| Nalco | 1 Tropfen |
| | /kg - |
| D-Biotin | 0,4 mg/kg |
| Thiamin.HCl | 2,0 mg/kg |
| CaCO₃ | 10 g/kg |

Die Versuche aus den Beispielen 2 und 3 wurden in Form einer Parallelfermentation durchgeführt. Dieser Begriff bezieht sich auf die Verwendung des gleichen Inokulummaterials(12 Kolben werden vereinigt, die erhaltene Kulturbrühe wird zu gleichen Teilen auf die Fermenter Bsp 2 und 3 verteilt) die Kontrolle von Temperatur, Drehzahl des Rührers, pO₂-Setpoint und Zufütterungsprofil für die Supplementlösung.

### Beispiel 2

### Fermentation im Standardreaktor:

Der Standardreaktor, der mit Rühr- und Temperiersystem ausgestattet ist, wurde mit 20 l des folgenden Mediums beschickt und 30 min bei 121 °C sterilisiert.

| | |
|---|---|
| Saccharose | 20 g/kg |
| Melasse | 5,3 g/kg |
| Maiskleberhydrolysat | 20 ml/kg |
| NH₄)₂SO₄ | 10 g/kg |
| Mg-Sulfat . 7H₂O | 0,75 g/kg |
| Citronensäure.H₂O | 0,6 g/kg |
| FeSulfat.7H₂O | 30 mg/kg |
| MnSulfat . H₂O | 30 mg/kg |
| Zn-Sulfat H₂O | 1,5 mg/kg |
| CaCl₂.2H₂ | 20 mg/kg |
| H₃PO₄ 89 % | 1,75 ml/kg |
| Biotin | 0,3 mg/kg |
| Thiamin.HCl | 0,2 mg/kg |
| Nalco | 1 g/kg |
| mit NH₄OH 25 % auf pH | |
| 7,5 v. Sterilisation | |
| pH 7.0 | |

Nach Abkühlung auf 30 °C wird der Fermenter mit 2 Liter des Inokulums beimpft. Die Kultur wird bei 30 °C und 400 rpm gerührt, der pO₂-Wert wird über die Belüftung (0,5-2 vvm) auf 20 % Sättigung geregelt. Der pH-Wert wird mittels 25 %-iger Ammoniaklösung auf pH 7 geregelt. Bei Schaumanfall wird nach Bedarf Struktol zur chemischen Schaumbekämpfung zugegeben. Nach Abfall der Saccharosekonzentration unter 10 g/l werden 2 sterile Lösungen zudosiert:
Zulaufmedium Zucker: 9,5 kg Lösung mit 500 g/kg Saccharose
Zulaufmedium Supplemente: 5 kg Lösung mit folgender
Zusammensetzung:

| | |
|---|---|
| Melasse | 18 g/kg |
| Maiskleberhydrolysat | 50 g/kg |
| (NH₄)₂SO₄ | 10 g/kg |
| Mg-sulfat.7H₂O | 0,375 g/kg |
| H₃PO₄ (89 %) | 0,9 g/kg |
| Citronensäure.H₂O | 0,6 g/kg |
| Fe-Sulfat .7H₂O | 15 mg/kg |
| Mn-Sulfat .H₂O | 15 mg/kg |
| Zn-Sulfat .7H₂O | 1,5 mg/kg |
| CaCl₂.2H₂O | 10 mg/kg |
| Biotin | 0,3 mg/kg |
| Thiamin HCl | 0,2 mg/kg |
| Nalco | 1 ml/kg |
| pH 7.5 vor Sterilisation | |

Die Zuckerzufütterung erfolgte so, daß im Reaktor immer eine Zuckerkonzentration > 10 g/l vorlag.
Die Supplementlösung wurde innerhalb 49 h kontinuierlich zugefüttert.
Nach 53 h Kultivationsdauer war die Fermentation beendet. Bis Kultivationsende wurden 4480 g Saccharose verbraucht und 1021 g Lys.HCl = 817 g Lysinbase gebildet. Die Gesamtbiotrockenmasse lag bei 327 g.

### Beispiel 3

### Fermentation im abgeänderten Standardreaktor

Der Reaktor wurde mit dem gleichen Medium wie unter Beispiel 2 beschrieben, befüllt und sterilisiert. Nach Einstellen der Fermentationsparameter wie dort wurde der Fermenter mit 2 Litern aus der Inokulumsschiene gemäß Beispiel 1 beimpft. Die Fermentationsführung (Temperatur, Drehzahl, pO₂-Setpoint, Zufütterungsprofil für Supplementlösung) entspricht dem des parallel betriebenen Standardreaktors.
Nach 53 h Kultivationsdauer wurde die Fermentation beeendet.
Bis zu diesem Zeitpunkt wurden 3852 g Saccharose verbraucht und 902 g Lys.HCl oder 722 g Lysinbase gebildet. Die Gesamttrockenmasse lag bei 305 g.

Die Parallelfermentationen zeigen, daß durch die Einbauten im Versuchsreaktor, die zu einer in großen Reaktoren üblichen Mischzeit führen, die Stoffumsätze sehr deutlich (um ca. 14 %), die Biotrockenmasse merklich (um ca. 7 %) reduziert wurde. Diese Informationen sind bedeutsam für das Scale up eines Produktionsprozesses.
Durch die gewählte Ausstattung des Versuchsreaktors kann somit sehr effizient und kostengünstig das Verhalten eines Fermentationsprozesses in großen Reaktoren simuliert werden. Dadurch ist es möglich, auf das kostenintensive Anpassen des Prozesses in Fermentern zunehmender Größe zu verzichten und sogleich die Prozeßauslegung an die Bedingungen bei einer Mischzeit von θ₉₀ von ca. 130 s anzupassen, wie sie für Reaktoren von ca. 100m³ und mehr Volumen gefunden wird.

Ein Bild des bevorzugt eingesetzten Versuchsreaktors findet sich in Abbildung 1.

Dabei haben die Ziffern folgende Bedeutung:
1.obere pH-Elektrode
2.obere pO₂-Elektrode
3.Scheibenrührer (insgesamt sechs Rührer)
4.Lochscheibe (insgesamt fünf Lochscheiben)
5.untere pH-Elektrode
6.untere pO₂-Elektrode
7.Begasungsring
8.Zufütterungsrohre

## Patentansprüche

1. Verwendung eines mit einem oder mehreren Scheibenrührern ausgestatteten Rührreaktors mit einem Volumen von 10 bis 500l, in dem zumindest eine Lochscheibe ober- und/oder unterhalb des(der) Scheibenrührer(s) horizontal angebracht ist und in dem sich das Verhältnis von Lochoberfläche zur Oberfläche der Lochscheiben auf 10 bis 40% beläuft, zur Ermittlung der Mischzeit θ₉₀.

2. Verwendung gemäß Anspruch 1,
**dadurch gekennzeichnet,**
**daß** zwei Lochscheiben zwischen zwei benachbarten Scheibenrührern horizontal angebracht sind.

3. Verwendung gemäß den Ansprüchen 1 oder 2,
**dadurch gekennzeichnet,**
**daß** die Lochscheiben an der Reaktorwand befestigt sind und keinen Kontakt zur Rührachse besitzen.

4. Verwendung gemäß den Ansprüchen 1 oder 2,
**dadurch gekennzeichnet,**
**daß** die Lochscheiben an der Rührachse befestigt sind.

5. Verwendung gemäß den Ansprüchen 1 oder 2,
**dadurch gekennzeichnet,**
**daß** die Lochscheiben abwechselnd an der Reaktorwand und an der Rührachse angebracht sind.

6. Verwendung gemäß den Ansprüchen 1 bis 5,
**dadurch gekennzeichnet,**
**daß** die Zahl der Scheibenrührer 2 bis 10 und die der Lochscheiben 1 bis 11 beträgt.

7. Verwendung gemäß den Ansprüchen 1 bis 6,
**dadurch gekennzeichnet,**
**daß** man die Mischzeit θ₉₀ für einen chemischen mikrobiellen oder enzymatischen Prozeß ermittelt.

## Claims

1. Use of an agitated reactor equipped with one or more disk agitators and of a volume of 10 to 500 l, in which at least one perforated disk is fitted horizontally above and/or below the disk agitator(s) and in which the ratio of the area of the perforations to the area of the perforated disks is 10 to 40% to determine the mixing time θ₉₀.

2. Use according to claim 1,
**characterised in that**
two perforated disks are fitted horizontally between two adjacent disk agitators.

3. Use according to claim 1 or 2,
**characterised in that**
the perforated disks are attached to the reactor wall and have no contact with the agitator shaft.

4. Use according to claim 1 or 2,
**characterised in that**
the perforated disks are attached to the agitator shaft.

5. Use according to claim 1 or 2,
**characterised in that**
the perforated disks are fitted alternately on the reactor wall and the agitator shaft.

6. Use according to claims 1 to 5,
**characterised in that**
the number of disk agitators is 2 to 10 and the number of perforated disks 1 to 11.

7. Use according to claims 1 to 6,
**characterised in that**
the mixing time θ₉₀ is determined for a chemical microbial or enzymatic process.

## Revendications

1. Utilisation d'un réacteur agité, équipé d'un ou de plusieurs agitateurs à disque, présentant un volume de 10 à 500 litres, dans lequel est disposé horizontalement au moins un disque perforé au-dessus et/ou au-dessous du ou des agitateurs à disque et dans lequel le rapport surface des perforations à surface du disque perforé est de 10 à 40 %, pour la détermination du temps de mélange θ₉₀.

2. Utilisation selon la revendication 1, **caractérisée en ce qu'**on a disposé horizontalement deux disques perforés entre deux agitateurs à disque voisins.

3. Utilisation selon les revendications 1 ou 2, **caractérisée en ce que** les disques perforés sont fixés sur la paroi du réacteur et ne sont pas en contact avec l'axe d'agitation.

4. Utilisation selon les revendications 1 ou 2, **caractérisée en ce que** les disques perforés sont fixés sur l'axe d'agitation.

5. Utilisation selon les revendications 1 ou 2, **caractérisée en ce que** les disques perforés sont disposés alternativement sur la paroi du réacteur et sur l'axe d'agitation.

6. Utilisation selon les revendications 1 à 5, **caractérisée en ce que** le nombre d'agitateurs à disque est de 2 à 10 et le nombre des disques perforés est de 1 à 11.

7. Utilisation selon les revendications 1 à 6, **caractérisée en ce qu'**on détermine le temps de mélange θ₉₀ pour un processus chimique microbien ou enzymatique.
